(19) Europäisches Patentamt
European Patent Office
Office européen des brevets

(11) Veröffentlichungsnummer: **0 088 282**
**B1**

(12) # EUROPÄISCHE PATENTSCHRIFT

(45) Veröffentlichungstag der Patentschrift: **04.12.85**

(51) Int. Cl.⁴: **C 07 C 143/822**, A 61 K 31/18

(21) Anmeldenummer: **83101693.6**

(22) Anmeldetag: **22.02.83**

(54) **Neue Indanyl-Derivate, ihre Herstellung und Verwendung.**

(30) Priorität: **04.03.82 DE 3208079**

(43) Veröffentlichungstag der Anmeldung: **14.09.83 Patentblatt 83/37**

(45) Bekanntmachung des Hinweises auf die Patenterteilung: **04.12.85 Patentblatt 85/49**

(84) Benannte Vertragsstaaten: **AT BE CH DE FR GB IT LI LU NL SE**

(56) Entgegenhaltungen:
**EP-A-0 009 554**
**EP-A-0 056 956**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Rufer, Clemens, Dr., Westhofener Weg 14, D-1000 Berlin 38 (DE)**
Erfinder: **Böttcher, Irmgard, Dr., Frobenstrasse 46, CH-4000 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

## Beschreibung

Die Erfindung betrifft neue Indanyl-Derivate der allgemeinen Formel I

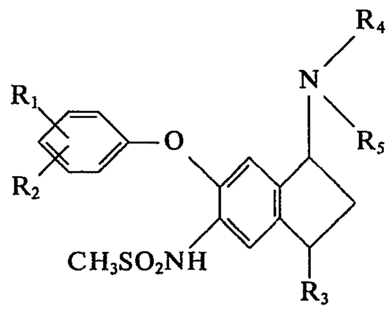

(I)

worin

$R_1$ und
$R_2$ gleich oder verschieden sind und jeweils ein Wasserstoffatom, ein Fluoratom oder ein Chloratom bedeuten,
$R_3$ ein Wasserstoffatom oder einen Phenylrest darstellt,
$R_4$ ein Wasserstoffatom oder einen 1 bis 6 Kohlenstoffatome enthaltenden Alkylrest bedeutet und
$R_5$ ein Wasserstoffatom, einen 1 bis 6 Kohlenstoffatome enthaltenden Alkylrest, einen Cyclohexylrest oder einen Phenylrest darstellt, mit der Maßgabe, daß mindestens einer der Substituenten $R_3$, $R_4$ und $R_5$ von Wasserstoff verschieden ist, und deren Salze mit physiologisch unbedenklichen Säuren.

Indanyl-Derivate der allgemeinen Formel I sind vorzugsweise solche, deren Substituenten $R_1$ und $R_2$ ein ortho- und ein para-ständiges Fluoratom darstellen, Indanyl-Derivate der allgemeinen Formel I, deren Substituent $R_3$ ein Wasserstoffatom bedeutet, Indanyl-Derivate der allgemeinen Formel I, deren Substituent $R_4$ ein Wasserstoffatom darstellt und Indanyl-Derivate der allgemeinen Formel I, deren Substituent $R_5$ ein Alkylrest ist.

Die Substituenten $R_1$ und $R_2$ der Indanyl-Derivate können gleichartig oder verschieden sein.

Weiterhin betrifft die Erfindung ein Verfahren zur Herstellung von Indanyl-Derivaten der allgemeinen Formel I, welches dadurch gekennzeichnet ist, daß man

a) ein Indan-Derivat der allgemeinen Formel II

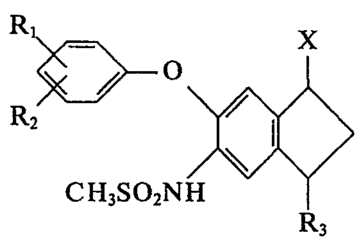

(II)

worin

$R_1$, $R_2$ und
$R_3$ die im Anspruch 1 genannte Bedeutung besitzen und
X ein Chloratom, ein Bromatom oder einen Arylsulfonylrest (insbesondere einen p-Toluolsulfonylrest) darstellt,

mit einem Amin der allgemeinen Formel III

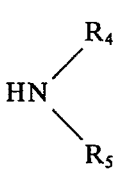

(III)

worin $R_4$ und $R_5$ die im Anspruch 1 genannte Bedeutung besitzen, kondensiert oder

b) ein Indan-Derivat der allgemeinen Formel IV

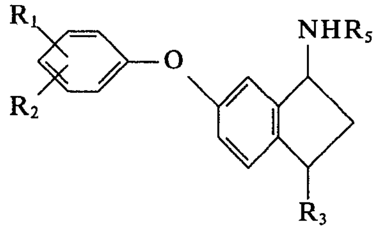

(IV)

hydriert
und gewünschtenfalls die erhaltenen Indan-Derivate in ihre Salze überführt.

Ein geeignetes Herstellungsverfahren ist beispielsweise das in »Methoden der Org. Chemie« (Houben–Weyl), Band XI/1, 4. Auflage 1957, Seite 24ff., Seite 217ff. und Seite 341ff. beschriebene Verfahren.
Die erfindungsgemäßen Verbindungen zeichnen sich durch eine gute antiphlogistische Wirksamkeit aus.
Darüber hinaus zeichnen sich die erfindungsgemäßen Substanzen durch eine analgetische, antidysmenorrhöische, antipyretische thrombocytenaggregationshemmende und diuretische Wirksamkeit aus. Darüber hinaus ist bemerkenswert, daß diese Substanzen die Prostaglandinsynthese kaum hemmen. Ein besonderer Vorzug dieser Verbindungen ist die große Dissoziation zwischen therapeutischer Wirksamkeit und unerwünschter Nebenwirkung (insbesondere Ulcerogenese).
Somit eignen sich die neuen Verbindungen in Kombination mit den in der galenischen Pharmazie üblichen Trägermitteln zur Behandlung von Erkrankungen des rheumatischen Formenkreises (wie der rheumatischen Arthritis, Ostearthritis oder ankylosierenden Spondalitis), Asthma bronchiale, Heufieber u. a.
Bemerkenswert ist ferner, daß sich die Indanyl-Derivate der allgemeinen Formel I gemäß Anspruch 1 darüber hinaus auch zur Behandlung von Migräne und von Dysmenorhoe eignen und das Thromboserisiko mindern.
Überraschenderweise gibt es unter den erfindungsgemäßen Indanyl-Derivaten auch solche, die zusätzlich noch eine ausgeprägte antiulcerogene sowie tumorhemmende Wirksamkeit besitzen.
Die Herstellung der Arzneimittelspezialitäten erfolgt in üblicher Weise, indem man die Wirkstoffe mit geeigneten Zusätzen, Trägersubstanzen und Geschmackskorrigentien in die gewünschten Applikationsformen wie Tabletten, Dragees, Kapseln, Lösungen, Inhalationsmitteln usw. überführt.
Für die orale Anwendung eignen sich insbesondere Tabletten, Dragees und Kapseln, welche beispielsweise 1 bis 250 mg Wirkstoff und 50 mg bis 2 g pharmakologisch unwirksamen Trägers, wie zum Beispiel Laktose, Amylose, Talkum, Gelatine, Magnesiumstearat und ähnliches, sowie die üblichen Zusätze enthalten.
Die Ausgangsverbindungen für das erfindungsgemäße Verfahren gemäß Anspruch 17 sind bekannt oder können in an sich bekannter Weise hergestellt werden.
Die nachfolgenden Ausführungsbeispiele dienen zur Erläuterung des erfindungsgemäßen Verfahrens.

Beispiel 1

Zu einer Suspension von 3,53 g 6-(2,4-Difluorphenoxy)-5-methylsulfonylamino-1-indanon in 40 ml Methanol werden 2 g Methylamin gefügt. Die entstehende Lösung wird zweieinhalb Stunden gerührt, danach werden 600 mg Palladium-Kohle (10%ig) hinzugefügt, und es wird unter Normaldruck hydriert. Nach Abfiltrieren des Katalysators wird eingeengt und aus einem Gemisch Ethanol—wäßrige Salzsäure umkristallisiert. 2,90 g N-[6-(2,4-Difluorphenoxy)-1-methylamino-5-indanyl]-methansulfonamid, Hydrochlorid vom Schmelzpunkt 221°C werden erhalten.
Analog Beispiel 1 wurden hergestellt:

Beispiel 2

N-[6-(2,4-Difluorphenoxy)-1-ethylamino-5-indanyl]-methansulfonamid, Hydrochlorid vom Schmelzpunkt 216°C.

Beispiel 3

N-[6-(3-Chlorphenoxy)-1-ethylamino-5-indanyl]-methansulfonamid, Hydrochlorid vom Schmelzpunkt 201°C.

Beispiel 4

N-[6-(2,4-Difluorphenoxy)-1-propylamino-5-indanyl]-methansulfonamid, Hydrochlorid vom Schmelzpunkt 210°C.

Beispiel 5

N-(6-Phenoxy-1-propylamino-5-indanyl)-methansulfonamid, Hydrochlorid vom Schmelzpunkt 197°C.

Beispiel 6

N-[1-Cyclohexylamino-6-(2,4-difluorphenoxy)-5-indanyl]-methansulfonamid, Hydrochlorid vom Schmelzpunkt 221°C.

Beispiel 7

N-[1-Anilino-6-(2,4-difluorphenoxy)-5-indanyl]-methansulfonamid vom Schmelzpunkt 129°C. In diesem Fall wurde nach Abfiltrieren des Katalysators eingeengt und mit Toluol über Kieselgel chromatographiert.

Beispiel 8

N-[6-(2,4-Difluorphenoxy)-3-phenyl-1-propylamino-5-indanyl]-methansulfonamid vom Schmelzpunkt 127°C.

Das für die Herstellung dieser Verbindung notwendige 6-(2,4-Difluorphenoxy)-5-methylsulfonylamino-3-phenyl-1-indanon wird auf folgendem Wege erhalten:

120 g 6-(2,4-Difluorphenoxy)-5-indanylamin werden 1,2 Liter Essigsäure und 0,5 Liter Essigsäureanhydrid 30 Minuten bei 50°C gehalten, anschließend wird bei dieser Temperatur eine Lösung von Chromtrioxid (110 g) in 50 ml Wasser und 300 ml Essigsäure eingetropft. Nach 30 Minuten bei 50°C wird eingeengt, Wasser zugesetzt, mit Chloroform extrahiert und mit Chloroform über eine Kieselgelsäure chromatographiert. 30 g 6-Acetylamino-5-(2,4-difluorphenoxy)-1-indanon vom Schmelzpunkt 210°C werden erhalten. Diese werden in 500 ml Tetrahydrofuran gelöst, und die Lösung wird bei 40°C mit einer Phenylmagnesiumbromidlösung in Ether (hergestellt aus 24 g Magnesium, 108 ml Brombenzol und 500 ml Ether) versetzt. Nach 16 Stunden bei 20°C wird mit Ammoniumchloridlösung zersetzt. Die organische Phase wird mit 8 g p-Toluolsulfonsäure 5 Minuten gerührt. Nach Filtration wird eingeengt, der Rückstand mit Benzin ausgerührt und aus Ethanol umkristallisiert. 22 g N-[6-(2,4-Difluorphenoxy)-3-phenyl-5-indenyl]-acetamid werden erhalten. Diese werden in 60 ml Dioxan und 240 ml Ethanol gelöst und über 1,2 g Palladium-Kohle (10%ig) hydriert. Nach Einengen und Kristallisation aus Benzin werden 16,5 g N-[6-(2,4-Difluorphenoxy)-3-phenyl-5-indanyl]-acetamid erhalten (Schmelzpunkt: 117°C). Diese werden in 200 ml Aceton mit einer Lösung von 8 g Chromtrioxid, 20 ml Schwefelsäure und 40 ml Essigsäure 16 Stunden bei 20°C gerührt. Nach Zugabe von Natriumhydrogensulfit wird eingeengt, in Chloroform aufgenommen, die Chloroformlösung mit Natronlauge gewaschen und eingeengt. Chromatographie über Kieselgel mit Toluol—Essigsäureethylester ergibt 6 g 5-Acetylamino-6-(2,4-difluorphenoxy)-3-phenyl-1-indanon vom Schmelzpunkt 163°C. Diese werden in 50 ml Ethanol und 10 ml Salzsäure (10normal) 2 Stunden gekocht. Nach Einengen wird mit Ammoniak schwach alkalisch gestellt, abgesaugt, getrocknet, in 60 ml Pyridin aufgenommen und mit 2,3 g Methansulfonylchlorid versetzt. Nach 25 Stunden bei 20°C wird eingeengt, mit Wasser versetzt, abgesaugt, in 1 n-Natronlauge gelöst und mit 10 n-Salzsäure angesäuert. Absaugen ergibt 3,1 g 6-(2,4-Difluorphenoxy)-5-methylsulfonylamino-3-phenyl-1-indanon vom Schmelzpunkt 101°C.

Beispiel 9

2,84 g N-[6-(2,4-Difluorphenoxy)-1-hydroxy-5-indanyl]-methansulfonamid und 1,83 g Phosphorpentachlorid werden in 50 ml Dichlormethan—Diethylether (1 : 1) 45 Minuten bei 0°C gerührt. Bei 0°C wird eine Lösung von 7 g Dimethylamin in 25 ml Dichlormethan hinzugefügt. Nach 2 Stunden bei 20°C und 20 Minuten Siedens wird eingeengt und der Rückstand aus wäßriger Salzsäure umkristallisiert. 2,41 g N-[6-(2,4-Diflourphenoxy)-1-dimethylamino-5-indanyl]-methansulfonamid, Hydrochlorid vom Schmelzpunkt 195°C werden erhalten.

Analog Beispiel 9 wurde hergestellt:

Beispiel 10

N-[6-(2,4-Difluorphenoxy)-1-dipropylamino-3-phenyl-5-indanyl]-methansulfonamid, Hydrochlorid vom Schmelzpunkt 167°C. Das für die Herstellung dieser Verbindung notwendige N-[6-(2,4-Difluorphenoxy)-1-hydroxy-3-phenyl-5-indanyl]-methansulfonamid wird auf folgenden Wege erhalten:

2,13 g 6-(2,4-Difluorphenoxy)-5-methylsulfonylamino-3-phenyl-1-indanon werden in 20 ml Methanol und 5,5 ml 1 n-Natronlauge gelöst. Bei 0°C werden 400 mg Natriumborhydrid eingetragen. Nach 16 Stunden bei 20°C wird eingeengt, mit Wasser versetzt, mit 1 n-Schwefelsäure neutralisiert

und abgesaugt. 1,7 g N-[6-(2,4-Difluorphenoxy)-1-hydroxy-5-indanyl]-methansulfonamid vom Schmelzpunkt 119°C werden erhalten.

**Patentansprüche**

1. Indanyl-Derivate der allgemeinen Formel I

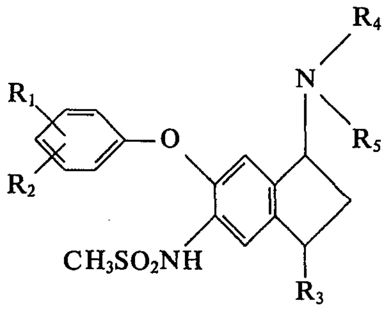

(I)

worin

$R_1$ und
$R_2$ gleich oder verschieden sind und jeweils ein Wasserstoffatom, ein Fluoratom oder ein Chloratom bedeuten,
$R_3$ ein Wasserstoffatom oder einen Phenylrest darstellt,
$R_4$ ein Wasserstoffatom oder einen 1 bis 6 Kohlenstoffatome enthaltenden Alkylrest bedeutet und
$R_5$ ein Wasserstoffatom, einen 1 bis 6 Kohlenstoffatome enthaltenden Alkylrest, einen Cyclohexylrest oder einen Phenylrest darstellt, mit der Maßgabe, daß mindestens einer der Substituenten $R_3$, $R_4$ und $R_5$ von Wasserstoff verschieden ist, und deren Salze mit physiologisch unbedenklichen Säuren.

2. Indanyl-Derivate der allgemeinen Formel I gemäß Patentanspruch 1, dadurch gekennzeichnet, daß die Substituenten $R_1$ und $R_2$ ein ortho- und ein paraständiges Fluoratom darstellen.
3. Indanyl-Derivate der allgemeinen Formel I gemäß Patentanspruch 1 und 2, dadurch gekennzeichnet, daß der Substituent $R_3$ ein Wasserstoffatom darstellt.
4. Indanyl-Derivate der allgemeinen Formel I gemäß Patentanspruch 1 bis 3, dadurch gekennzeichnet, daß der Substituent $R_4$ ein Wasserstoffatom darstellt.
5. Indanyl-Derivate der allgemeinen Formel I gemäß Patentanspruch 1 bis 4, dadurch gekennzeichnet, daß der Substituent $R_5$ einen Alkylrest darstellt.
6. N-[6-(2,4-Difluorphenoxy)-1-methylamino-5-indanyl]-methansulfonamid.
7. N-[6-(2,4-Difluorphenoxy)-1-ethylamino-5-indanyl]-methansulfonamid.
8. N-[6-(2,4-Difluorphenoxy)-1-propylamino-5-indanyl]-methansulfonamid.
9. N-(6-Phenoxy-1-propylamino-5-indanyl]-methansulfonamid.
10. N-[1-Cyclohexylamino-6-(2,4-difluorphenoxy)-5-indanyl]-methansulfonamid.
11. N-[6-(2,4-Difluorphenoxy)-3-phenyl-1-propylamino-5-indanyl]-methansulfonamid.
12. N-[6-(2,4-Difluorphenoxy)-1-dimethylamino-5-indanyl]-methansulfonamid.
13. N-[6-(2,4-Difluorphenoxy)-1-dipropylamino-3-phenyl-5-indanyl]-methansulfonamid.
14. N-[6-(3-Chlorphenoxy)-1-ethylamino-5-indanyl]-methansulfonamid.
15. N-[1-Anilino-6-(2,4-difluorphenoxy)-5-indanyl]-methansulfonamid.
16. Pharmazeutische Präparate, gekennzeichnet durch einen Gehalt an einem Indanyl-Derivat gemäß Patentanspruch 1 bis 15.
17. Verfahren zur Herstellung von Indanyl-Derivaten der allgemeinen Formel I gemäß Patentanspruch 1, dadurch gekennzeichnet, daß man

a) ein Indan-Derivat der allgemeinen Formel II

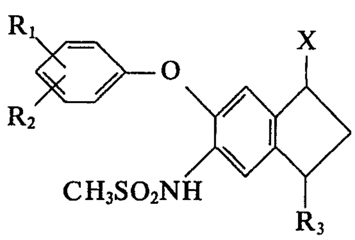

(II)

worin

$R_1$, $R_2$ und
$R_3$ die im Anspruch 1 genannte Bedeutung besitzen und
X ein Chloratom, ein Bromatom oder einen Arylsulfonylrest (insbesondere einen p-Toluolsulfonylrest) darstellt

mit einem Amin der allgemeinen Formel III

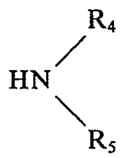

(III)

worin $R_4$ und $R_5$ die im Anspruch 1 genannte Bedeutung besitzen, kondensiert oder

b) ein Indan-Derivat der allgemeinen Formel IV

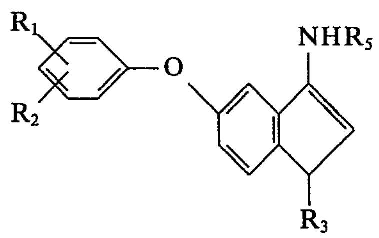

(IV)

hydriert
und gewünschtenfalls die erhaltenen Indan-Derivate in ihre Salze überführt.

**Claims**

1. Indanyl derivatives of general formula I

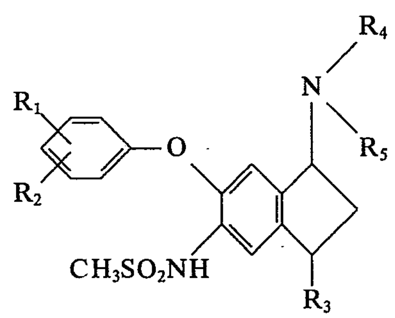

(I)

wherein

$R_1$ and
$R_2$ are the same or different and each represents a hydrogen atom, a fluorine atom or a chlorine atom,
$R_3$ represents a hydrogen atom or a phenyl group,
$R_4$ represents a hydrogen atom or an alkyl group of 1 to 6 carbon atoms,
$R_5$ represents a hydrogen atom, an alkyl group of 1 to 6 carbon atoms, a cyclohexyl group or a phenyl group, with the proviso that at least one of the substituents $R_3$, $R_4$ and $R_5$ is hydrogen, and their salts with physiologically acceptable acids.

2. Indanyl derivatives of the general formula I according to claim 1, characterised in that the substituents $R_1$ and $R_2$ represent an ortho- and a para-position fluorine atom.
3. Indanyl derivatives of the general formula I according to claims 1 and 2, characterised in that the substituent $R_3$ represents a hydrogen atom.
4. Indanyl derivatives of the general formula I according to claims 1 to 3, characterised in that the substituent $R_4$ represents a hydrogen atom.
5. Indanyl derivatives of the general formula I according to claims 1 to 4, characterised in that the substituent $R_5$ represents an alkyl group.
6. N-[6-(2,4-difluorophenoxy)-1-methylamino-5-indanyl]-methanesulphonamide.

7. N-[6-(2,4-difluorophenoxy)-1-ethylamino-5-indanyl]-methanesulphonamide.
8. N-[6-(2,4-difluorophenoxy)-1-propylamino-5-indanyl]-methanesulphonamide.
9. N-[6-phenoxy-1-propylamino-5-indanyl]-methanesulphonamide.
10. N-[1-cyclohexylamino-6-(2,4-difluorophenoxy)-5-indanyl]-methanesulphonamide.
11. N-[6-(2,4-difluorophenoxy)-3-phenyl-1-propylamino-5-indanyl]-methanesulphonamide.
12. N-[6-(2,4-difluorophenoxy)-1-dimethylamino-5-indanyl]-methanesulphonamide.
13. N-[6-(2,4-difluorophenoxy)-1-dipropylamino-3-phenyl-5-indanyl]-methanesulphonamide.
14. N-[6-(3-chlorophenoxy)-1-ethylamino-5-indanyl]-methanesulphonamide.
15. N-[1-anilino-6-(2,4-difluorophenoxy)-5-indanyl]-methanesulfonamide.
16. Pharmaceutical preparations characterised by a content of an indanyl derivative according to claims 1 to 15.
17. Process for the preparation of indanyl derivatives according to claim 1 characterised in that one
a) condenses an indane derivative of the general formula II

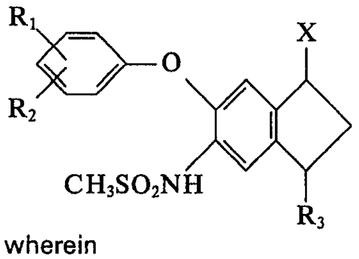

(II)

wherein

$R_1$, $R_2$ and
$R_3$ have the meanings given in claim 1 and
X represents a chlorine atom, a bromine atom or an arylsulphonyl group (especially a p-toluenesulphonyl group)

with an amine of the general formula III

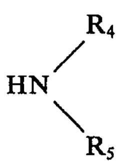

(III)

wherein $R_4$ and $R_5$ have the meaning given in claim 1, or
b) hydrogenates an indane derivative of the general formula IV

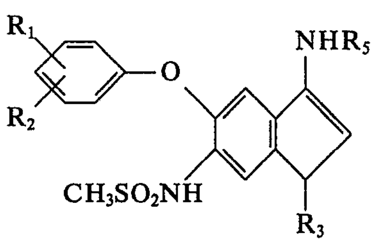

(IV)

and converts if desired the obtained indane derivatives into their salts.

**Revendications**

1. Dérivés de l'indane de formule générale I cidessous:

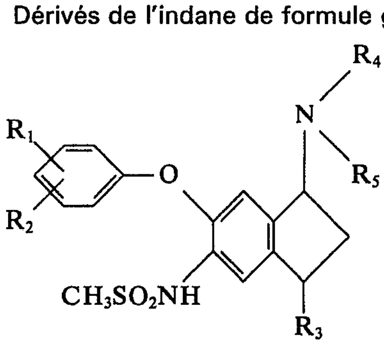

(I)

dans laquelle

$R_1$ et

$R_2$, qui peuvent être identiques ou différents l'un de l'autre, représentent chacun un atome d'hydrogène, de fluor ou de chlore,

$R_3$ est un atome d'hydrogène ou un radical phényle,

$R_4$ est un atome d'hydrogène ou un alkyle en $C_1$–$C_6$, et

$R_5$ est un atome d'hydrogène, un alkyle en $C_1$–$C_6$, un cyclohexyle ou un phényle, avec le condition que parmi $R_3$, $R_4$ et $R_5$, l'un d'eux au moins ne soit pas l'hydrogène, ainsi que leurs sels formés avec des acides sans inconvénient physiologique.

2. Dérivés de l'indane de formule I selon la revendication 1 dans lesquels $R_1$ et $R_2$ sont des atomes de fluor aux positions ortho et para.

3. Dérivés selon la revendication 1 ou 2 dans lesquels $R_3$ est un atome d'hydrogène.

4. Dérivés selon l'une quelconque des revendications 1 à 3 dans lesquels $R_4$ est un atome d'hydrogène.

5. Dérivés selon l'une quelconque des revendications 1 à 4 dans lesquels $R_3$ est un radical alkyle.

6. N-[6-(2,4-Difluorophénoxy)-1-méthylamino-5-indanyl]-méthanesulfonamide.

7. N-[6-(2,4-Difluorophénoxy)-1-éthylamino-5-indanyl]-méthanesulfonamide.

8. N-[6-(2,4-Difluorophénoxy)-1-propylamino-5-indanyl]-méthanesulfonamide.

9. N-(6-Phénoxy-1-propylamino-5-indanyl)-méthanesulfonamide.

10. N-[1-Cyclohexylamino-6-(2,4-difluorophénoxy)-5-indanyl]-méthanesulfonamide.

11. N-[6-(2,4-Difluorophénoxy)-3-phényl-1-propyl-amino-5-indanyl]-méthanesulfonamide.

12. N-[6-(2,4-Difluorophénoxy)-1-diméthylamino-5-indanyl]-méthanesulfonamide.

13. N-[6-(2,4-Difluorophénoxy)-1-dipropylamino-3-phényl-5-indanyl]-méthanesulfonamide.

14. N-[6-(3-Chlorophénoxy)-1-éthylamino-5-indanyl]-méthanesulfonamide.

15. N-[1-Anilino-6-(2,4-difluorophénoxy)-5-indanyl]-méthanesulfonamide.

16. Compositions pharmaceutiques contenant un dérivé de l'indane selon l'une quelconque des revendications 1 à 15.

17. Procédé de préparations de dérivés d'indane de formule générale I selon la revendication 1, procédé caractérisé en ce que, ou bien

a) on condense un dérivé de l'indane de formule générale II

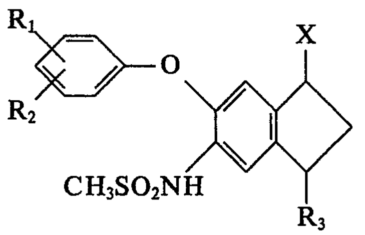

(II)

dans laquelle

$R_1$, $R_2$ et

$R_3$ ont les significations données à la revendication 1 et

X désigne un atome de chlore ou de brone ou un radical arène-sulfonyle (notament p-toluène-sulfonyle),

avec une amine de formule générale III

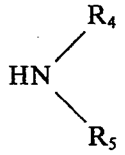

(III)

dans laquelle

$R_4$ et $R_5$ ont les significations données à la revendication 1, ou bien

b) on hydrogene un dérivé de l'indane de formule générale IV

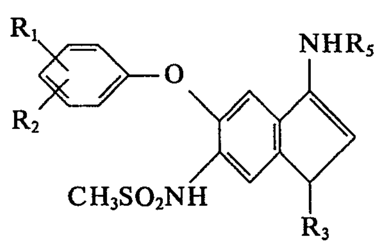

(IV)

et, si l'on veut, on transforme en sels les dérivés d'indane ainsi obtenus.